# EUROPEAN PATENT SPECIFICATION

(11) **EP 3 130 372 B1**
(45) Date of publication and mention of the grant of the patent: **08.01.2020**
(21) Application number: 16177043.3
(22) Date of filing: 30.06.2016
(51) Int. Cl.: A61M 25/00

(54) **CATHETER AND BALLOON CATHETER**
KATHETER UND BALLONKATHETER
CATHÉTER ET CATHÉTER À BALLONNET

(30) Priority: 10.08.2015 JP 2015157962
(43) Date of publication of application: 15.02.2017
(73) Proprietor: ASAHI INTECC CO., LTD., Seto-shi, Aichi 489-0071 (JP)
(72) Inventor: KUBO, Yuta, Seto-shi, Aichi 489-0071 (JP); KATSURADA, Takeharu, Seto-shi, Aichi 489-0071 (JP); OGIDO, Moritaka, Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 1 721 631
- EP-A1- 2 762 187
- WO-A1-2014/189828
- JP-A- H07 323 090
- JP-A- 2012 249 811

## Description

### TECHNICAL FIELD

The present invention relates to a catheter and a balloon catheter for medical use.

### BACKGROUND

As a conventional catheter, for example, a configuration as disclosed in JP 2012-100829 A has already been proposed comprising: an elongated tubular main body including an inner layer, a coil layer (a reinforcement body) formed with a wire material wound around the outer periphery of the inner layer and an outer layer covering the coil body.

Further, JP 3659664 B discloses a medical tube comprising an inner layer, a reinforcement layer configured with a coil layer including a first coil and a second coil adhering to the outside of the inner layer and an outer layer adhering to the outside of the reinforcement layer.

However, the catheter according to JP 2012-100829 A and the medical tube according to JP 3659664 B described above have the following problem: a coil as a reinforcement body tends to expand due to the springback when bent, resulting in a lift of an end of the coil.

Moreover, as a configuration which may solve the above problem, JP 2012-249812 A and JP 2012-249811 A disclose an outer catheter comprising: an inner layer, an outer layer and an intermediate layer (a reinforcement body) formed with a linear object and provided so as to be sandwiched between the inner layer and the outer layer, wherein the distal end of the intermediate layer is covered with an contrasting ring (a protective layer).

### SUMMARY OF THE INVENTION

### TECHNICAL PROBLEM

However, in the case of the outer catheter according to JP 2012-249812 A and JP 2012-249811 A, the outer diameter of the catheter is disadvantageously larger at an end of the intermediate layer because the contrasting ring is thick.

Accordingly, an objective of the present invention is to provide a catheter and a balloon catheter in which a lift of a reinforcement body can be prevented without increasing the outer diameter at an end of the reinforcement body.

### SOLUTION TO PROBLEM

The present invention provides a catheter comprising: a tubular inner layer, a reinforcement body wound around the outer periphery of the inner layer, a protective layer partially covering the outer periphery of the reinforcement body, and an outer layer covering at least the outer periphery of the protective layer, wherein the protective layer covers at least the outer periphery of one end of the reinforcement body, and the one end of the reinforcement body covered with the protective layer is buried deeper in a side of the inner layer relative to a portion of the reinforcement body not covered with the protective layer.

In the aforementioned configuration, a lift of an end of the reinforcement body when bent can be prevented because the end of the reinforcement body is covered with the protective layer. Further, the end of the reinforcement body covered with the protective layer is buried and constrained in the inner layer. Therefore, a relative movement to the inner layer is controlled as compared with an unburied portion of the reinforcement body, and a lift when bent can be prevented more reliably. Furthermore, the end of the reinforcement body is buried in the inner layer, and thus the outer diameter of the whole catheter at that portion can be prevented from becoming larger than those of other portions.

In addition, the protective layer is desirably configured with a resin.

A catheter having the aforementioned configuration can easily be manufactured and processed. In addition, breakage of a catheter can be prevented in general if a low stiffness resin is used for the protective layer.

Further, the protective layer is desirably configured with a heat-shrinkable resin tube.

The manufacturing process can be simplified when the protective layer is configured with a so-called heat-shrinkable resin tube according to the aforementioned configuration.

In addition, preferably, the outer layer is configured with a resin, and the resin of the protective layer has a higher melting point than that of the outer layer.

According to the aforementioned configuration, the protective layer can be prevented from becoming thinner when the outer layer is fused to the inner layer. Therefore, the strength of the protective layer can be assured, and a risk of a lifted end of the reinforcement body can be prevented even more reliably.

In addition, the protective layer desirably covers both the one end and the other end of the reinforcement body.

According to the aforementioned configuration, a lift of the reinforcement body from the inner layer due to the springback can be prevented even more reliably, and the outer diameters at one end and the other end of the reinforcement body can also be prevented from becoming larger.

Further, a balloon catheter according to the present invention comprises the above catheter and a balloon joined to the outer periphery of the outer layer in the above catheter.

According to the aforementioned configuration, a balloon catheter can be provided having a structure in which a lift of an end of the reinforcement body can be prevented.

### ADVANTAGEOUS EFFECTS OF THE INVENTION

The present invention can provide a catheter in which a lift of an end of a reinforcement body can be prevented without increasing the outer diameter at the end of the reinforcement body.

Further, the present invention can provide a balloon catheter having a structure in which a lift of an end of a reinforcement body can be prevented without increasing the outer diameter at the end of the reinforcement body.

### BRIEF DESCRIPTION OF THE DRAWINGS

Fig. 1 shows a top view of the catheter according to Example 1.
Fig. 2 shows an enlarged partial cross sectional view of the catheter according to Example 1.
Figs. 3A to 3C show a schematic diagram illustrating a manufacturing process of the catheter according to Example 1.
Fig. 4 shows an enlarged partial cross sectional view of the catheter according to Example 2.
Fig. 5 shows an enlarged partial cross sectional view of the catheter according to Example 3.
Fig. 6 shows a partial cross sectional top view of the balloon catheter according to Example 4.
Fig. 7 shows an enlarged partial cross sectional view of the balloon catheter according to Example 4.
Fig. 8 shows an enlarged partial cross sectional view of the balloon catheter according to Example 5.

### DESCRIPTION OF EMBODIMENTS

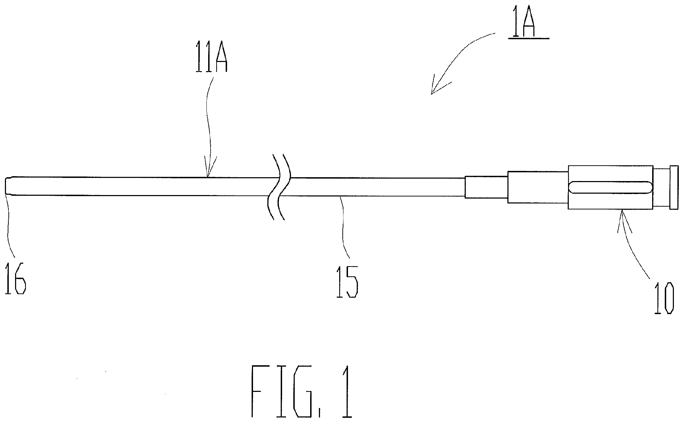

Below, Examples in which the catheters according to the present invention are embodied will be described in detail. However, the present invention shall not be limited to Examples shown below, and modifications in design can be made appropriately. References to embodiments throughout the description which are not under the scope of the appended claims merely represent possible exemplary executions and are therefore not part of the present invention. In Figs. 1 to 8, the left side corresponds to the distal end side which is to be inserted into the body, and the right side corresponds to the proximal side which is to be operated by an operator such as a physician.

### [Example 1]

As shown in Fig. 1, a catheter device 1A comprises a catheter 11A, and the catheter 11A has an operating portion 10 to be operated by an operator, a main body 15 extending from the operating portion 10 and a distal end part 16 located at the distal end side of the main body 15.

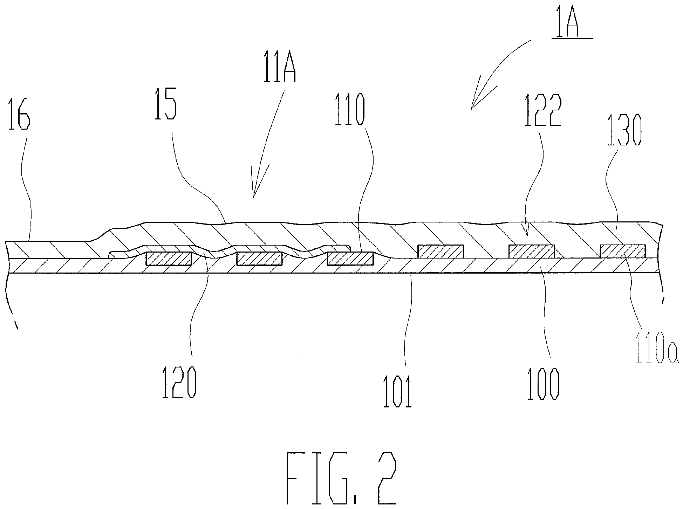

More specifically, the catheter 11A has a tubular inner layer 100 as shown in Fig. 2. The inner layer 100 comprises an inner lumen 101 through which a guide wire or the like is to be inserted.

Further, a coil body 110 is disposed at the outer periphery of the inner layer 100 as a reinforcement body in which a wire 110a is spirally wound.

Moreover, a protective layer 120 is formed on the outer periphery of a distal end part corresponding to one end of the coil body 110 such that the distal end part of the coil body 110 is covered with the protective layer 120. Note that in the proximal end side relative to the distal end part of the coil body 110, an unprotected portion 122 is formed which is not covered with the protective layer 120. Therefore, the protective layer 120 partially covers the outer periphery of the coil body 110.

Further, a portion of the coil body 110 covered with the protective layer 120 is in a state where the wire 110a of the coil body 110 is buried in the inner layer 100 toward the side of the inner layer 100. In contrast, the wire 110a of the coil body 110 at the unprotected portion 122 is not buried in the inner layer 100.

In addition, an outer layer 130 is formed on the outer peripheries of the inner layer 100, the coil body 110 and the protective layer 120, and the inner layer 100, the coil body 110 and the protective layer 120 are covered with the outer layer 130.

Further, a coating layer covering the outer layer 130 may be provided on the outer periphery of the outer layer 130.

Note that the distal end part 16 of the catheter 11A is constituted of a part of the inner layer 100, a part of the coil body 110, the protective layer 120, a part of the unprotected portion 122 and a part of the outer layer 130.

Note that the inner layer 100 preferably comprises a resin such as polyethylene, polyurethane, polyamide, polyamide elastomer, polyolefine, polyester or polyester elastomer. Further, in view of slideability of a guide wire and the like to be inserted into the inner lumen 101, the inner periphery of the inner layer 100 may be coated with a fluorine-based resin such as PEFE.

Moreover, the coil body 110 preferably comprises stainless steel (SUS 304), tungsten or a superelastic alloy such as a Ni-Ti alloy. Note that the cross sectional shape of the wire 110a of the coil body 110 is not limited to a rectangular shape, and it may be a circular shape, an elliptical shape, a polygonal shape or the like.

Further, the protective layer 120 desirably comprises a heat-shrinkable resin tube having a melting point higher than the outer layer 130. Preferred are, for example, a tubes comprising polyethylene terephthalate resin (PET resin), polyimide resin, or polyether ether ketone resin (PEEK resin). This can prevent the protective layer 120 from becoming thinner when fused, in a case where the outer layer 130 is fused to the inner layer 100. As a result, the strength of the protective layer 120 can be assured, and a risk of a lifted end of the coil body 110 can be prevented. Note that the protective layer 120 may comprise a metal material, but preferably comprises a resin in view of easier manufacturing and processing of the catheter 11A. Further, breakage of the catheter 11A can be prevented in general if a low stiffness resin is used for the protective layer 120.

Moreover, the outer layer 130 desirably comprises a resin such as polyethylene, polyurethane, polyamide, polyamide elastomer, polyolefine, polyester or polyester elastomer.

According to the catheter 11A, a lift of the coil body 110 from the inner layer 100 due to the springback can be prevented by virtue of the protective layer 120 when the catheter 11A is bent. Further, the distal end part of the coil body 110 covered with the protective layer 120 is buried in the inner layer 100, and the periphery of the wire 110a of the coil body 110 at that portion is covered with the inner layer 100. Therefore, the distal end part of the coil body 110 is constrained by the inner layer 100, and more resistant to a lift of the distal end part of the coil body 110 when bent. Furthermore, the catheter 11A can be prevented from having a larger outer diameter because the distal end part of the coil body 110 is buried in the inner layer 100.

In addition, the flexibility as an elastic body is not impaired because the wire 110a of the coil body 110 at the unprotected portion 122 is not buried in the inner layer 100. Compared with a case where the full length of the coil body 110 is buried in the inner layer 100 without providing the unprotected portion 122, the catheter 11A can more easily bend along a blood vessel or the digestive organ. As a result, the catheter 11A is more resistant to breakage on the way when inserted into a blood vessel or the digestive organ.

Note that the wire 110a of the coil body 110 may be completely buried in the inner layer 100, or may be partially buried in the inner layer 100.

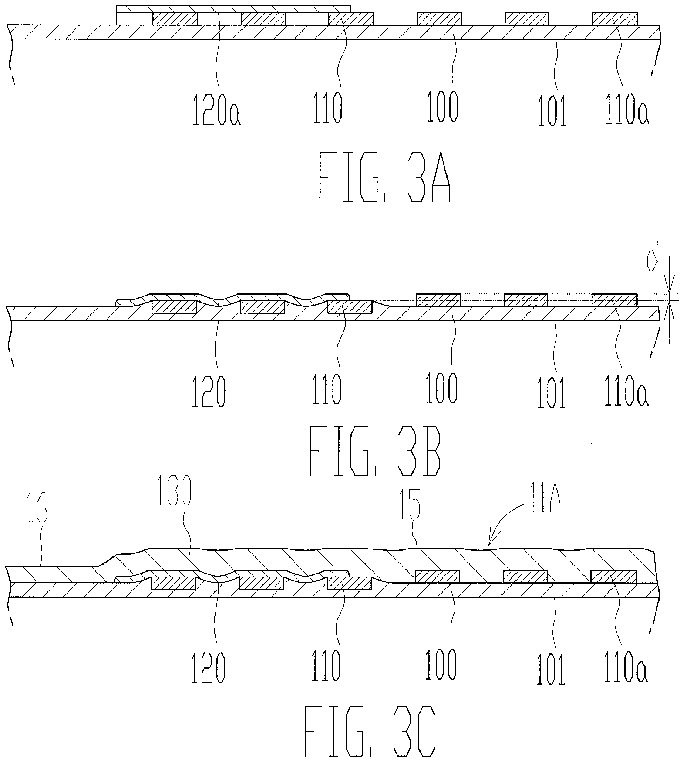

Below, a method for manufacturing the catheter 11A will be described briefly. First, as shown in Fig. 3A, the coil body 110 is wound around the outer periphery of the inner layer 100, and a heat-shrinkable resin tube 120a is further placed on the outer periphery of the distal end part of the coil body 110.

Next, the heat-shrinkable resin tube 120a is heated and shrunk to form the protective layer 120, and at the same time, the wire 110a of the distal end part of the coil body 110 is pressed against the side of the inner layer 100 to be buried by a depth, d, as shown in Fig. 3B.

Then, the outer layer 130 is fused to the outer peripheries of the inner layer 100, the coil body 110 and the protective layer 120 as shown in Fig. 3C. Note that the outer layer 130 may be formed after performing thermal shrinkage of the heat-shrinkable resin tube to form the protective layer 120, or may be formed upon performing the thermal shrinkage.

The manufacturing process can be simplified in a case where the heat-shrinkable resin tube 120a is used for the protective layer 120 because the protective layer 120 can be formed simultaneously when the outer layer 130 is fused with the inner layer 100.

Note that the above manufacturing method is merely an example, and a different manufacturing method may be used, of course. For example, the protective layer 120 may be formed on the periphery of the coil body 110 after the coil body 110 is buried to the side of the inner layer 100 by the so-called swaging process. Alternatively, a tube comprising a resin material or a metal material as the protective layer 120 may be placed on the outer periphery of the distal end part of the coil body 110 instead of the heat-shrinkable nature tube 120a. Then, the wire 110a of the coil body 110 may be buried in the side of the inner layer 100 by the swaging process.

### [Example 2]

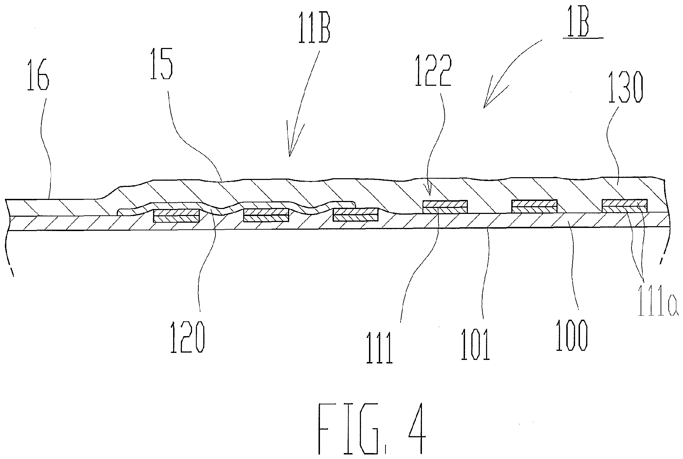

A catheter 11B according to Example 2 will be described with reference to Fig. 4. Note that the same reference numbers are assigned to structures similar to those in Example 1, and the descriptions thereof are omitted.

The catheter 11B in a catheter device 1B comprises a braid 111 in which two or more wires 111a are woven in a mesh-like manner instead of the coil body 110 in Example 1. The braid 111 serves as a reinforcement body. Note that the multiple wires 111a of the braid 111 preferably comprise stainless steel (SUS 304), tungsten or a superelastic alloy such as a Ni-Ti alloy. The cross sectional shapes of the two or more wires 111a of the braid 111 are not limited to be rectangular, and they may be circular, elliptical, polygonal or the like.

In addition, the distal end part of the braid 111 is covered with the protective layer 120, and further, the wires 111a of the braid 111 covered with the protective layer 120 are buried in the inner layer 100.

Again, according to the aforementioned configuration, a lift of the braid 111 from the inner layer 100 due to the springback can be prevented by virtue of the protective layer 120 when the catheter 11B is bent. Further, the distal end part of the braid 111 covered with the protective layer 120 is buried in the inner layer 100, and constrained by being covered with the inner layer 100. Therefore, a lift of the braid 111 can be even further prevented when bent. Moreover, the catheter 11B can be prevented from having a large outer diameter because the braid 111 is buried in the inner layer 100. Further, the flexibility as an elastic body is not impaired because the wires 111a of the braid 111 at the unprotected portion 122 are not buried in the inner layer 100. The catheter 11B can more easily bend along a blood vessel or the digestive organ as compared with a case where the full length of the braid 111 is buried in the inner layer 100 without forming the unprotected portion 122. As a result, the catheter 11B is more resistant to breakage on the way when the catheter 11B is inserted into a blood vessel or the digestive organ.

### [Example 3]

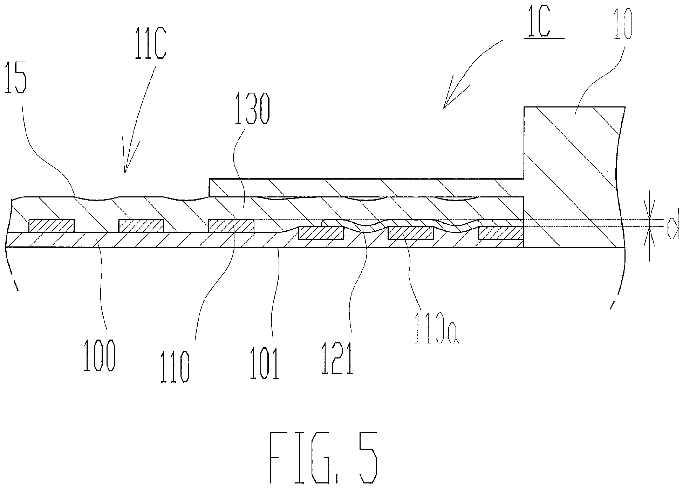

A catheter 11C according to Example 3 will be described with reference to Fig. 5. Note that the same reference numbers are assigned to structures similar to those in Examples 1 and 2, and the descriptions thereof are omitted.

In the catheter 11C used for a catheter device 1C, a protective layer 121 is also formed at a proximal end part, which corresponds to the other end, in addition to the distal end part of the coil body 110. Further, the proximal end part of the coil body 110 is buried in the side of the inner layer 100 by a depth, d, using the protective layer 121.

According to the aforementioned configuration, a lift of the coil body 110 can be prevented at the distal end part and the proximal end part of the coil body 110. Further, the distal end part and the proximal end part of the coil body 110 can be prevented from having large outer diameters.

### [Example 4]

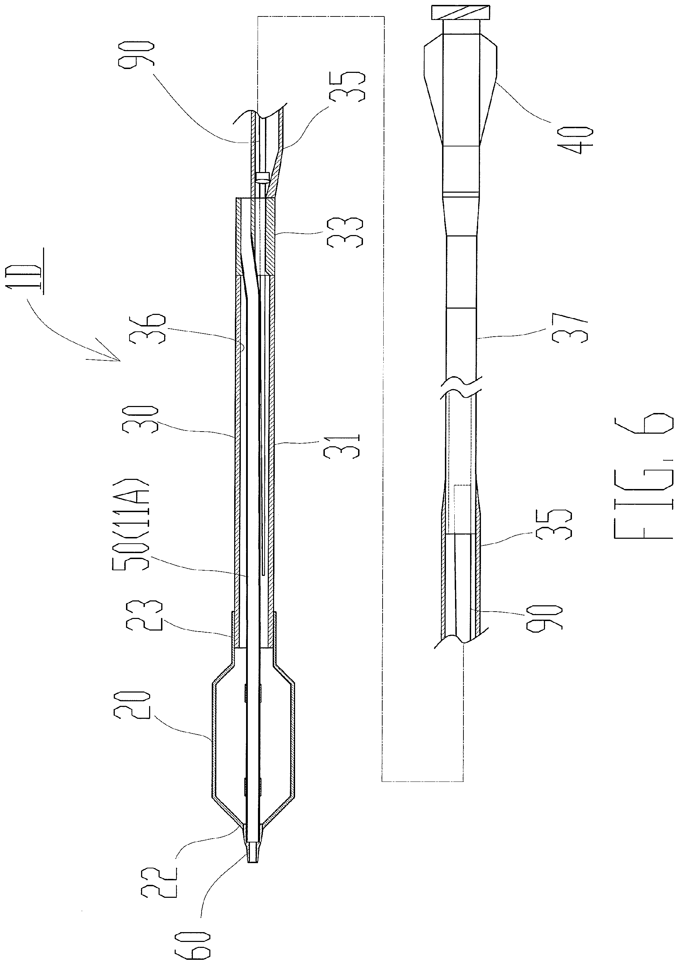

A balloon catheter 1D according to Example 4 will be described with reference to Fig. 6. Note that the same reference numbers are assigned to structures similar to those in Examples 1 to 3, and the descriptions thereof are omitted.

The balloon catheter 1D used for treating a stenosis site, for example, in a blood vessel of the heart comprises a balloon 20. The balloon catheter 1D further comprises an outer shaft 30, a connector 40, an inner shaft 50 corresponding to the catheter 11A, a tip 60 and a core wire 90.

The balloon 20 has a function to expand a stenosis site, and comprises a resin material. The balloon 20 further has a distal end attachment part 22 at the distal end side, and a proximal end attachment part 23 at the proximal end side. The distal end attachment part 22 is joined to the distal end of the inner shaft 50 through the tip 60, and the proximal end attachment part 23 is joined to the distal end of the outer shaft 30.

The outer shaft 30 has a function to supply a fluid, and comprises a tubular member constituting an inflation lumen 36 for supplying the fluid. Further, the outer shaft 30 has a distal end outer shaft portion 31, a guide wire port portion 33, a middle outer shaft portion 35 and a proximal end outer shaft portion 37 in the order from the distal end side. Note that the guide wire port portion 33 corresponds to a portion where the distal end outer shaft portion 31, the middle outer shaft portion 35 and the inner shaft 50 are joined.

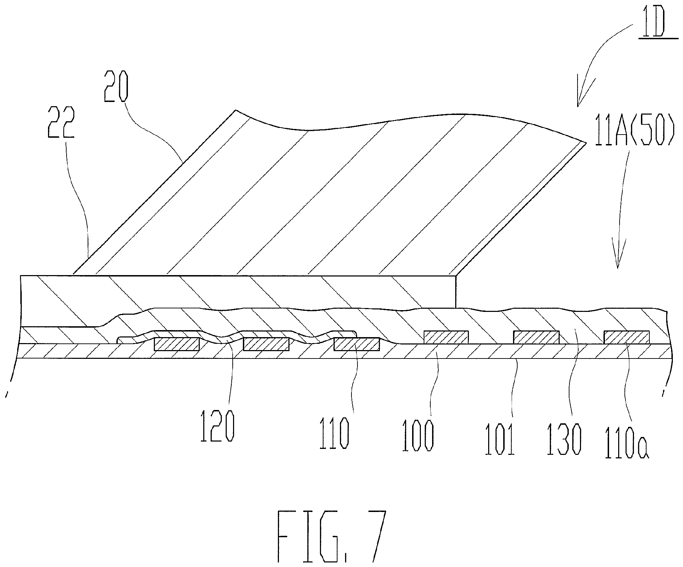

As shown in Fig. 7, the balloon catheter 1D in the aforementioned configuration has an inner shaft 50 which is the catheter 11A in which a lift of an end of the coil body 110 can be prevented. Therefore, a lift of an end of the coil body 110 can effectively be prevented, although the end of the coil body 110 might otherwise be caused by the pressure applied in the direction of the outer periphery due to a contrast agent, a saline solution or the like when a balloon 20 is inflated.

### [Example 5]

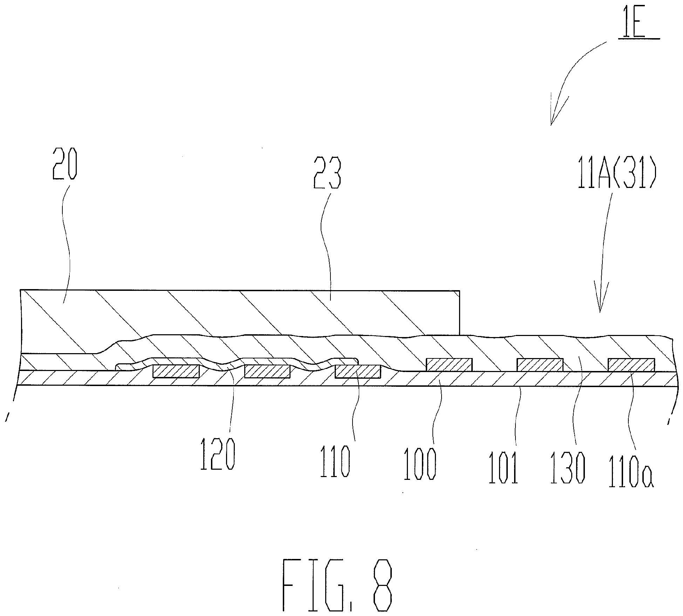

A balloon catheter 1E according to Example 5 will be described with reference to Fig. 8. Note that the same reference numbers are assigned to structures similar to those in Examples 1 to 4, and the descriptions thereof are omitted.

As shown in Fig. 8, the balloon catheter 1E comprises the catheter 11A according to Example 1 as the distal end outer shaft portion 31. Again, according to the aforementioned construction, a lift of an end portion of the coil body 110 can be prevented in the balloon catheter 1E.

Note that the present invention shall not be limited to the embodiments according to Examples 1 to 5, and a wide range of modifications in dimensions and shapes of components can be made appropriately.

Further, the catheter 11A according to Example 1 is used as the inner shaft 50 and the distal end outer shaft portion 31 in Examples 4 and 5, but the configurations shall not be limited to these. For example, the catheter 11B according to Example 2 may be used as the inner shaft 50 and the distal end outer shaft portion 31, or the catheter 11C according to Example 3 may be used as the inner shaft 50 and the distal end outer shaft portion 31.

Furthermore, in Examples 1 to 5 described above, the coil body 110 and the braid 111 as the reinforcement bodies are not buried in the inner layer 100 at all at the unprotected portion 122 which is not covered with the protective layer 120, but the configuration shall not be limited to this. At the unprotected portion 122, the coil body 110 and the braid 111 as the reinforcement bodies may be somewhat buried in the inner layer 100 to an extent that flexibility as an elastic body is not lost.

### DESCRIPTION OF SYMBOLS

- 1A, 1B, 1C: Catheter device
- 1D, 1E: Balloon catheter
- 11A, 11B, 11C: Catheter
- 20: Balloon
- 100: Inner layer
- 110: Coil body (reinforcement body)
- 111: Braid (reinforcement body)
- 120: Protective layer
- 122: Unprotected portion
- 130: Outer layer

## Claims

1. A catheter (11A, 11B, 11C) comprising:
a tubular inner layer (100);
a reinforcement body (110, 111) wound around an outer periphery of the inner layer (100);
a protective layer (120, 121) partially covering an outer periphery of the reinforcement body (110, 111); and
an outer layer (130) covering at least an outer periphery of the protective layer (120, 121),
wherein the protective layer (120, 121) covers at least an outer periphery of one end of the reinforcement body (110, 111),
**characterized in that**
the one end of the reinforcement body (110, 111) covered with the protective layer (120, 121) is buried deeper in a side of the inner layer (100) relative to a portion (122) of the reinforcement body (110, 111) not covered with the protective layer (120, 121).

2. The catheter (11 A, 11B, 11C) according to claim 1, wherein the protective layer (120, 121) comprises a resin.

3. The catheter (11 A, 11B, 11C) according to claim 1 or 2, wherein the protective layer (120, 121) comprises a heat-shrinkable resin tube.

4. The catheter (11 A, 11B, 11C) according to claim 2 or 3, wherein the outer layer (130) comprises a resin, and the resin of the protective layer (120, 121) has a melting point higher than that of the outer layer (130).

5. The catheter (11A, 11B, 11C) according to any one of claims 1 to 4, wherein the protective layer (120, 121) covers both the one end and the other end of the reinforcement body (110, 111).

6. The catheter (11A, 11C) according to any one of claims 1 to 5, wherein the reinforcement body (110) is configured by a coil body (110).

7. The catheter (11B) according to any one of claims 1 to 5, wherein the reinforcement body (111) is configured by a braid (111).

8. A balloon catheter (1D, 1E) comprising:
the catheter (11A, 11B, 11C) according to any one of claims 1 to 7, and
a balloon (20) joined to an outer periphery of the outer layer (130) in the catheter (11A, 11B, 11C).

## Patentansprüche

1. Katheter (11A, 11B, 11C) mit:
einer schlauchförmigen Innenschicht (100);
einem Bewehrungskörper (110, 111), der um einen Außenumfang der Innenschicht (100) gewickelt ist;
einer Schutzschicht (120, 121), die einen Außenumfang des Bewehrungskörpers (110, 111) teilweise überzieht; und
einer Außenschicht (130), die wenigstens einen Außenumfang der Schutzschicht (120, 121) überzieht,
wobei die Schutzschicht (120, 121) wenigstens einen Außenumfang eines Endes des Bewehrungskörpers (110, 111) überzieht,
**dadurch gekennzeichnet, dass**
das mit der Schutzschicht (120, 121) überzogene eine Ende des Bewehrungskörpers (110, 111) tiefer in der Seite der Innenschicht (100) eingegraben ist als ein nicht mit der Schutzschicht (120, 121) überzogener Teil (122) des Bewehrungskörpers (110, 111).

2. Katheter (11A, 11B, 11C) nach Anspruch 1, wobei die Schutzschicht (120, 121) ein Harz enthält.

3. Katheter (11A, 11B, 11C) nach Anspruch 1 oder 2, wobei die Schutzschicht (120, 121) einen Wärmeschrumpfschlauch umfasst.

4. Katheter (11A, 11B, 11C) nach Anspruch 2 oder 3, wobei die Außenschicht (130) ein Harz enthält, und das Harz der Schutzschicht (120, 121) einen höheren Schmelzpunkt hat als dasjenige der Außenschicht (130).

5. Katheter (11A, 11B, 11C) nach einem der Ansprüche 1 bis 4, wobei die Schutzschicht (120, 121) sowohl das eine Ende als auch das andere Ende des Bewehrungskörpers (110, 111) überzieht.

6. Katheter (11A, 11C) nach einem der Ansprüche 1 bis 5, wobei der Bewehrungskörper (110) aus einem Wicklungskörper (110) gebildet ist.

7. Katheter (11B) nach einem der Ansprüche 1 bis 5, wobei der Bewehrungskörper (111) aus einem Geflecht (111) gebildet ist.

8. Ballonkatheter (1D, 1E) mit:
einem Katheter (11A, 11B, 11C) nach einem der Ansprüche 1 bis 7, und
einem an einen Außenumfang der Außenschicht (130) des Katheters (11A, 11B, 11C) angefügten Ballon (20).

## Revendications

1. Cathéter (11A, 11B, 11C) comprenant :
une couche interne tubulaire (100) ;
un corps de renforcement (110, 111) enroulé autour d'une périphérie externe de la couche interne (100) ;
une couche protectrice (120, 121) couvrant partiellement une périphérie externe du corps de renforcement (110, 111) ; et
une couche externe (130) couvrant au moins une périphérie externe de la couche protectrice (120, 121),
où la couche protectrice (120, 121) couvre au moins une périphérie externe d'une extrémité du corps de renforcement (110, 111),
**caractérisé en ce que**
l'extrémité du corps de renforcement (110, 111) couverte par la couche protectrice (120, 121) est enfouie plus profondément dans un côté de la couche interne (100) qu'une partie (122) du corps de renforcement (110, 111) non couverte par la couche protectrice (120, 121).

2. Cathéter (11A, 11B, 11C) selon la revendication 1, où la couche protectrice (120, 121) comprend une résine.

3. Cathéter (11A, 11B, 11C) selon la revendication 1 ou 2, où la couche protectrice (120, 121) comprend un tube en résine thermo-rétractable.

4. Cathéter (11A, 11B, 11C) selon la revendication 2 ou 3, où la couche externe (130) comprend une résine, et la résine de la couche protectrice (120, 121) a un point de fusion plus élevé que celui de la couche externe (130).

5. Cathéter (11A, 11B, 11C) selon l'une quelconque des revendications 1 à 4, où la couche protectrice (120, 121) couvre une extrémité et l'autre extrémité du corps de renforcement (110, 111).

6. Cathéter (11A, 11C) selon l'une quelconque des revendications 1 à 5, où le corps de renforcement (110) est configuré par un corps bobiné (110).

7. Cathéter (11B) selon l'une quelconque des revendications 1 à 5, où le corps de renforcement (111) est configuré par une tresse (111).

8. Cathéter à ballonnet (1D, 1E) comprenant :
le cathéter (11A, 11B, 11C) selon l'une quelconque des revendications 1 à 7, et
un ballonnet (20) joint à une périphérie externe de la couche externe (130) dans le cathéter (11A, 11B, 11C).
